# EUROPEAN PATENT APPLICATION

(11) **EP 3 231 474 A1**
(43) Date of publication of application: **18.10.2017**
(21) Application number: 17162559.3
(22) Date of filing: 23.03.2017
(51) Int. Cl.: A61N 1/02, A61N 1/04, A61N 1/36, A61N 1/375

(54) **PROCESS FOR ESTABLISHING A MUSCULAR ELECTROSTIMULATION PROTOCOL, AND RESPECTIVE PORTABLE MUSCULAR ELECTROSTIMULATION EQUIPMENT USING SAID PROTOCOL**

(30) Priority: 31.03.2016 BR 102016007240
(71) Applicant: Medecell S.A., Montevideo (UY)
(72) Inventor: Marques de Oliveira, Mauricio, São Paulo (BR); Manso, Willians Morales, São Paulo (BR); Bighetti, Moacyr Ramos, São Paulo (BR)
(74) Representative: Michalski Hüttermann & Partner Patentanwälte mbB

(57) **Abstract**

Said process and equipment being used in muscular performance recovery in patients in general, more specifically, in the ones practicing physical activity and athletes, the process providing the serial application of electrical pulses, transcutaneously, whose protocol involves the following stimulation steps: vasodilation phase, recovery phase and untightening phase; the equipment (1) consists of a monolithic cabinet (2) within which there is an electronic module (3), consisting of a body formed by two halves (3a) and (3b), between which a printed circuit board (4) is mounted, said board carrying the electronic circuit and the microcontroller; a power supply consisting of at least one battery (5) is placed inside the cabinet (2), whose external lower face houses a main electrode (6), and, extending from one of the distal sides thereof there are two electric conductor cables (7), at whose ends there are two satellite electrodes (8), said three electrodes (6) and (8) being endowed, on one of their faces, of self-adhesive gel plates; an adjustable strip (9) placed in the external lower face of the cabinet (2) can be used to secure the equipment (1) around the upper and lower limbs of the patient or athlete; in the external upper face of the cabinet (2), there is a control panel (10) with a power button (11) and two intensity selection buttons (12). The following internal components are also provided: Power source module (13), step-up regulator module (14), micro controller module (15), power supply seal module (16), boost source module (18), intensity regulation module (17), H-bridge module (19), and electrode output module (20).

## Description

### TECHNICAL FIELD

The present patent relates to a process for establishing a muscular electrostimulation protocol and a portable muscular electrostimulation equipment using said protocol, said process and equipment being used to recover the user's muscular performance in general, and more specifically, of practitioners of physical activity and athlets.

This innovative process and equipment makes possible its use in muscular recovery of users in the field, in the physical or sports practice sites, with a friendly and intuitive interface, and an innovative electro-stimulation protocol that accelerates the muscle recovery of physical activity practitioners.

### DESCRIPTION OF THE STATE OF THE ART

The use of electrical stimulation is well-established in the medical and sports practice as a valuable therapeutic resource and auxiliary resource for physical conditioning. Different stimulation parameters produce different biological effects, such as the control of hyperalgesia of different etiologies, muscular hypertrophy of normal and denervated muscles, increase in muscular resistance and strenght, and untightening and muscular relaxation.

As for the performance of physical activities, the involved musculature gradually loses its function, in a way that is directly proportional to the intensity and duration of the exercise. Hence, the depletion of the muscle energy stock, the accumulation of lactic acid and contraction occur.

The application of electrical stimuli from 7-10 Hz promotes capillar vasodilation in muscles and increases blood flow in ulcers. Lower frequencies has an untightening effect and promote endorphin release. Electrical stimulation associated with special diet increases the energy income and accelerates the reposition of muscular glycogen reserves. The concept of active muscular recovery via neural consists in mild exercise following a period of high intensity athletic performance.

Electrostimulation has shown an increase in blood flow and a recovery of muscular group. Electrostimulation has also proven to increase glucose capture, both by the sustained vasodilation and by muscle contraction itself.

In general terms, electrostimulation equipment can be divided into two large groups: bench equipment, powered by the electric grid, and portable ones, powered by batteries. For the use of bench equipment, it is necessary to move the user to the places where the appliance is available. On the contrary, in the case of portable equipment, the apparatus can be moved to application sites of the therapy, thus allowing field stimulation, for instance, in the sports practice sites.

Among the muscular electrostimulation equipment known in the prior art, let us mention the ones disclosed in documents N°s. US 4.832.033, US 6.507.757, US 8.565.888 and US 8.620.438.

A number of multiple-feature portable equipments are available in the market, operated through interfaces of variable complexity. These equipments are usually handled by experts (physiotherapists or coaches), without the athlete getting involved in the operation. Said equipments can apply several protocols of electrostimulation, but their sizes are still not very handy for use in the field. Furthermore, such equipments are difficult to set, as their interfaces are not user-friendly.

On the other hand, environmental conditions for the use of these equipments in sports practice are quite severe in terms of temperature, mechanical shocks and moisture. Moreover, currently available equipments have relatively low strength, with low resistance to high temperatures and shock, and are not watertight, being subject to moisture.

All of these factors constitute serious drawbacks of the equipment known in the art.

### PURPOSES OF THE INVENTION

To solve the drawbacks of ordinary equipments, the inventor(s) has developed an innovative muscular electrostimulation protocol, which provides the serial application of electrical stimuli with specific parameters to obtain increased muscular blood flow, the active recovery of the myocytes and stimulated musculature untightening.

This new protocol proved capable of significantly reversing the decrease in athletic performance due to muscle fatigue. This is an innovative way to promote muscle recovery, with great potential of use and market.

Some programmable bench equipment on the market could eventually be adapted to apply the innovative protocol developed herein. However, existing bench equipments involve complex operation restricted to trained professionals, and cannot be moved to outdoor environments where sports practice is carried out. On its turn, the portable equipment available in the market is not programmable like the bench ones, and, therefore, they cannot apply the innovative protocol developed herein. Therefore, the target public of this invention, that is, the practitioners of physical activities, would not be attended by any equipment known in the art, of any of the aforementioned categories.

Thus, the inventor(s) also devised a new portable muscular electrostimulation device capable of applying the developed electrostimulation protocol in a portable, battery-powered format, and with much better usability than the products found in the market.

Said equipment is self-applied and has a low cost, with a specific electronics and mechanical design for its use in physical activity and athletes in the anatomical regions corresponding to the large muscle groups intensively required in the various exercises and sports modalities. Such equipment enabled the use of this resource outside the outpatient environment or gyms, spreading its use to the field, in the environments in which the exercises, tests or matches are performed, both for professional athletes and for amateur athletes who aim to improve their performance.

The equipment in question further has, as an additional feature, a high degree of robustness, and can be used in extreme environmental conditions, which the usual equipment cannot tolerate. Thanks to the innovative solution of including analog digital circuits in monolithic blocks of polymers, the equipment developed herein is resistant to moisture and to electrical and mechanical shocks.

On the other hand, the equipment in question is quite ergonomic, as it has been developed to be used in the main anatomical regions of the major muscular groups involved in the practice of most sports exercises.

Furthermore, the portability of this novel equipment is higher than the one of the ordinary ones known in the art, its production cost is lower, and its production process is optimized, allowing its mass production with standardize quality, and providing a longer service life due to the fact that there is no contact of its sensitive areas with the external environment.

### DESCRIPTION OF THE DRAWINGS

To complement the present description, in order to better understand the characteristics of the subject matter of the patent, a set of drawings accompanies this specification, in which, in an exemplified and nonlimiting manner, the following has been represented:
- Figure 1 is a graph showing the ratio of mean voxel counts for each individual group of thigh muscles, between the right and left sides of an individual, a ratio obtained before and after the application of muscular electrostimulation according to the novel electrostimulation protocol, and using this innovative electrostimulation equipment;
- Figure 2 is a graphical representation of the largest electrostimulation cycle according to this novel electrostimulation protocol, and using this innovative electrostimulation equipment;
- Figures 3 and 4 show the equipment in question from top perspective and bottom perspective, respectively;
- Figure 5 is an exploded view of said equipment;
- Figure 6 shows the same through a partial and enlarged section;
- Figure 7 is a block diagram of said equipment, showing its internal electrical components;
- Figure 8 is the electrical scheme of this innovative equipment;
- finally, Figure 9 is a flowchart of the software, specifically developed for this innovative muscular electrostimulation protocol.

### DETAILED DESCRIPTION OF THE INVENTION

The present patent relates to a "PROCESS FOR ESTABLISIHING A MUSCULAR ELECTROSTIMULATION PROTOCOL, AND THE RESPECTIVE PORTABLE MUSCULAR ELECTROSTIMULATION EQUIPMENT USING SAID PROTOCOL", said process and equipment being used to recover the user's muscular performance in general, and more specifically, of practitioners of physical activity and athletes.

Initially, as for the "PROCESS FOR ESTABLISHING A MUSCULAR ELECTROSTIMULATION PROTOCOL", said process provides the serial application of electrical stimuli (or pulses), with specific parameters to obtain an increase in muscular blood flow, the active recovery of the myocytes and the untightening of the stimulated musculature.

To achieve this novel muscular electrostimulation protocol, quantitative kinetics studies, using digital motion capture equipment, were carried out in athletes with and without electrostimulation. Athletes undergoing electrostimulation showed a substantial improvement of the performance of different parameters, and a 50% power increase. Although there was an active muscle recovery identified in the improvement of the performance of athletes undergoing electrostimulation, it was necessary to use a methodology that objectively demonstrated this muscle recovery gain.

Muscle perfusion scintigraphy with SPECTICT, using methoxyisobutyl isonitrilised radiopharmacol with technetium-99m (sestamibi-^{99m}Tc) as a tracer, demonstrates differences in muscle perfusion, since it is routinely used to evaluate myocardial perfusion at rest and after exertion Physical or pharmacological.

Sestamibi-^{99m}Tc is commonly used to identify high celular metabolism tissues, to the extent that it was first used in myocardial scintigraphy. Sestamibi-^{99m}Tc 6 is a lipophilic cation concentrating in viable cells with high metabolism and high mitochondrial content through passive diffusion through the mitochondrial membrane, due to the transmembrane electrotransport potential of the cells. Thus, through pioneering experiments, the inventor was able to determine the ability of electrostimulation to increase blood flow and recovery of muscle groups through objective parameters, quantifying this gain through radioisotope imaging.

In the experimental phase, athletes were submitted to the following protocol of stimulation of the muscle groups of the right thigh for 25 minutes:
a) frequency of 8 Hz, which causes vasodilation, increasing muscle blood flow;
b) frequency of 30 Hz, which promotes active muscle recovery without recruiting type 1 fibers, of greater energetic consumption;
c) frequency of 2 Hz, which has untightening effect.

As type 1 fibers, which are responsible for resistance, have a high consumption of glycogen stores, they are expected to replenish their energy stocks more quickly than other muscle fibers. Moreover, a frequency of 2-8 Hz also promotes endorphin and encephalin release, which have an anxiolytic and analgesic effects, contributing to the athlete's psychological improvement.

Muscular perfusion scintigraphy with SPECTICT using the radio-drug sestaniibi-⁹⁹ⁿⁱTc was carried out in two moments: before electrostimulation and 20 minutes after electrostimulation of the athletes' tight. The same acquisition parameters were followed in both studies.

Image acquisition was performed through a two-head scintillation camera with SPECTICT (SYMBIA, SIEMENS). Regions of interest based on Voxels (VOls) were drawn manually in the rectus femoris muscles, vastus medialis and vastus laterallis bilaterally, before and after muscular electrostimulation.

The application of muscular electrostimulation was performed only on the right thigh, and then both thighs were compared.

The VOI design was based on CT anatomical data.

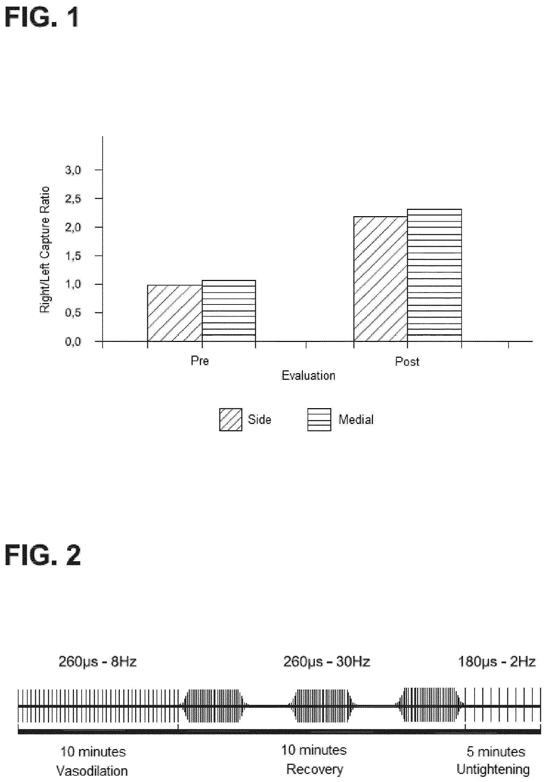

Attached Figure 1 shows an average counting ratio for each individual group of muscle (rectus femoris, vastus medialis and vastus laterallis) among the ones of the right and left sides. This ratio was obtained during rest and following muscular electrostimulation.

The results shown in the graph of said Figure 1 show a significant increase in sestamibi-^{99m}Tc capture following EMS, which indicates a great increase in muscle blood flow following electrostimulation. In absolute counts, this capture increase attained, in some cases, values 7 times higher following EMS.

A similar protocol could not be found either in scientific literature or in patent databases. This is an innovative mode to promote muscle recovery, with great use and market potential.

Therefore, upon manual regulation of initial stimulus intensity, until obtaining the optimal sufficient intensity to initiate muscular contraction, which can vary between OVp-p and 60Vp-p (with 500Ω charge), this innovative protocol involves the following steps:
- Step 1: Automatic application (via embedded software) of a first subcycle of electrical pulses, whose width ranges between 100 µs and 400 µs, preferably 260 µs, whose frequency ranges between 4 Hz and 12 Hz, preferably 8 Hz, and whose amplitude ranges between 0V and 100V (with 500Ω charge), preferably between 0V and 60V (with 500Ω charge), transcutaneously, for a period between 1 and 20 minutes, to obtain muscular vasodilation (vasodilation phase);
- Step 2: Automatic application (via embedded software) of a second subcycle of electrical pulses, consisting of pulse bursts, with pulses having width ranging between 100 µs and 400 ps, preferably 260 µs, with frequency ranging between 20 Hz and 40 Hz, preferably 30 Hz, and with amplitude ranging between 0V and 100V (with 500 Ω charge), preferably ranging between 0V and 60V (with 500Ω charge), during a period ranging from 1 to 20 minutes, also transcutaneously, for the differential mobilization of muscle fibers of low energy consumption, aiming to restore the stocks of energy substances in all muscle fibers, including the ones of high consumption (recovery phase); This subcycle involves an upward intensity ramp from 0V (with 500 Ω charge) to the value initially set by the user for 1 second, followed by a plateau maintaining that maximum amplitude for 4 seconds, followed by a ramp of downward intensity going from the value set by the user to 0V (with 500 Ω charge) for 1 second; a stimulation pause of preferably 4 seconds is inserted between each pulse burst, which are repeated several times, during a total from 1 to 20 minutes; and - Step 3: Automatic application (via embedded software) of a first subcycle of electrical pulses, whose width ranges between 100 µs and 250 µs, preferably 180 µs, whose frequency ranges between 1 Hz and 3 Hz, preferably 2 Hz, and whose amplitude ranges between 0V and 100V (with 500Ω charge), preferably between 0V and 60V (with 500 Ω charge), also transcutaneously, for a period between 1 and 5 minutes, to obtain musculature untightening (untightening phase);

At the end of the said three electrostimulation phases (Steps 1, 2 and 3), the automatic shutdown of the equipment occurs (via embedded software).

In the three electrostimulation phases of the process (Steps 1, 2 and 3), electrical pulses can have a square, trapezoidal or triangular shape, preferably trapezoidal.

Moreover, in the three electrostimulation phases of the process (Steps 1, 2 and 3), the electrical pulses can have a biphasic or monophasic morphology, preferably asymmetrical biphasic.

Moreover, according to this innovative "PROCESS FOR ESTABLISHING A MUSCULAR ELECTROSTIMULATION PROTOCOL", the three electrostimulation phases (Steps 1, 2 and 3) can exhibit different durations, wherein the long electrostimulation cycle, shown in Figure 2, contemplates the maximum durations disclosed for each one of the steps and total time. For use in various sports modalities, cycles and sub cycles with shorter durations are provided, optimized for each case of use.

Therefore, during the long electrostimulation cycle (Figure 2), Step 1 (vasodilation phase) lasts 10 minutes; Step 2 (recovery phase) lasts 10 minutes; and Step 3 (untightening phase) lasts 5 minutes.

Subcycles can be adapted according to the specific demands of the users within the diverse sports modalities, contemplating the differentiated needs of vasodilation, recovery and untightening. All this within the time limits provided by the duration of the tests, matches and training.

Depending on the need, this innovative process discloses the use of electrostimulation cycles in conjunction with dietary supplements, to obtain the maximum recovery effect from athletic performance.

Also depending on the need, this innovative process discloses the use of electrostimulation cycles in combination with drugs, to increase their tissue concentration in the stimulated regions of the body.

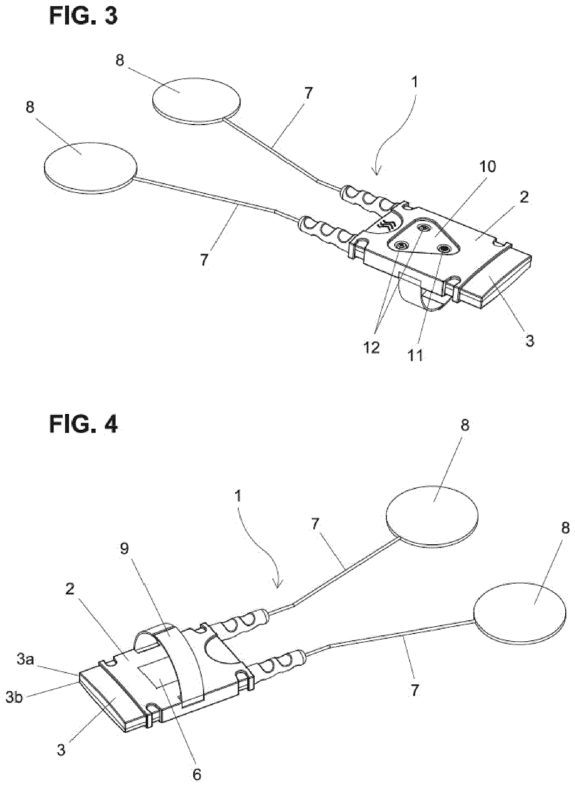

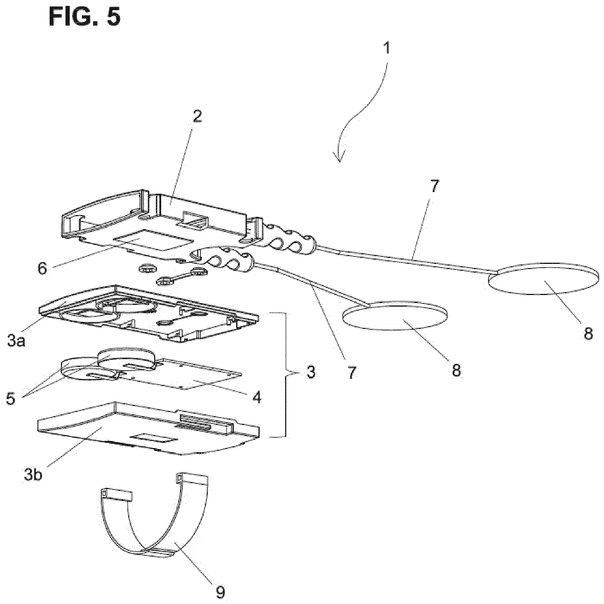

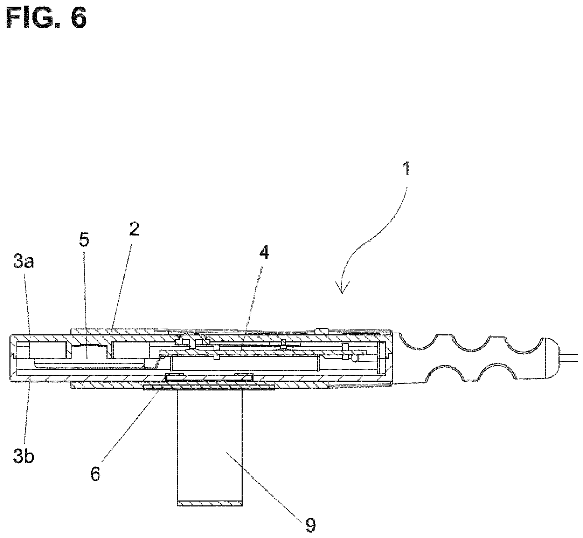

As for this innovative "PORTABLE MUSCULAR ELECTROSTIMULATION EQUIPMENT", said equipment is shown in details in attached Figures 3-6.

Such equipment (1) consists of a monolithic cabinet (2) substantially parallelepiped, watertight, molded in flexible polymer or silicone, within which there is an electronic module (3), consisting of a body having the same shape, also molded in a flexible polymer or silicone, formed by two watertight halves (3a) and (3b), between which there is a printed circuit board (4) with miniaturized technology, carrying the electronic circuit and the microcontroller responsible for the automated control of the stimulation parameters; between the halves (3a) and (3b) of the internal electronic module (3) there is also a power source consisting of one or more batteries (5), which can be nickel-cadmium (NiCd), nickel-metal hydride (NiMh) or lithium-ions (Li-Ion), batteries, preferably a lithium- ion (Li-Ion) one.

Said printed circuit board (4) uses the surface-mount technology (SMT).

In the external lower face of the cabinet (2), there is a main electrode (6), while, extending from one of the distal sides of said cabinet (2) there are two electric conductor cables (7), at whose ends there are two satellite electrodes (8), said three electrodes (6) and (8) having, on one of their faces, more specifically, on their face facing the body of the patient or athlete, self-adhesive gel plates, for application and fixation in the various regions of the body whose muscles will be electrostimulated.

An adjustable strip (9) extends from the non-distal edges of the external lower face of the cabinet (2), wherein said strap (9) can be used to secure the equipment (1) around the upper and lower limbs of the patient or athlete.

In the external upper face of the cabinet (2), there is a control panel (10) with a power button (11) and two intensity selection buttons (12) (one to increase and one to decrease the intensity).

Upon switching on the equipment, the user regulates the ideal intensity through said buttons (12), until there is sufficient intensity to initiate muscle contraction, which, as previously mentioned, varies from 0Vp-p to 60Vp-p (with 500Ω charge). At each "click" of intensity increase or reduction, a variation of 3V occurs (with 500Ω charge).

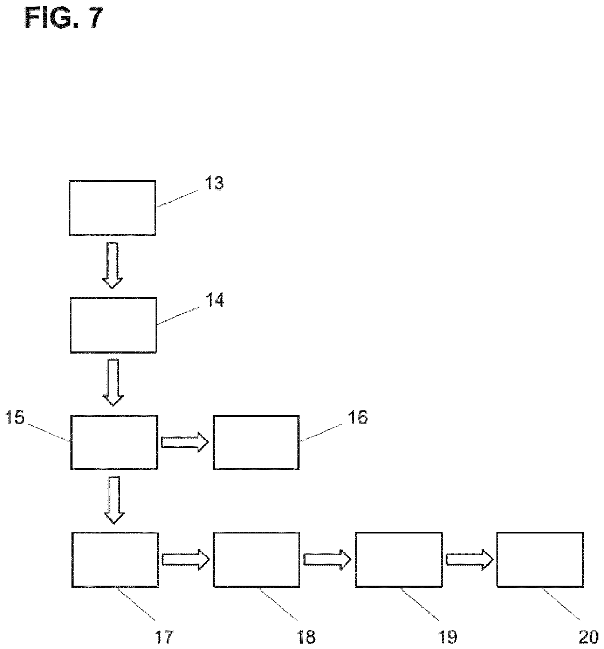

The block diagram of Figure 7 illustrates the internal components of the electronic module of this innovative equipment, namely: Power source module (13), step-up regulator module (14), micro controller module (15), power supply seal module (16), boost source module (18), H-bridge module (19), and electrode output module (20).

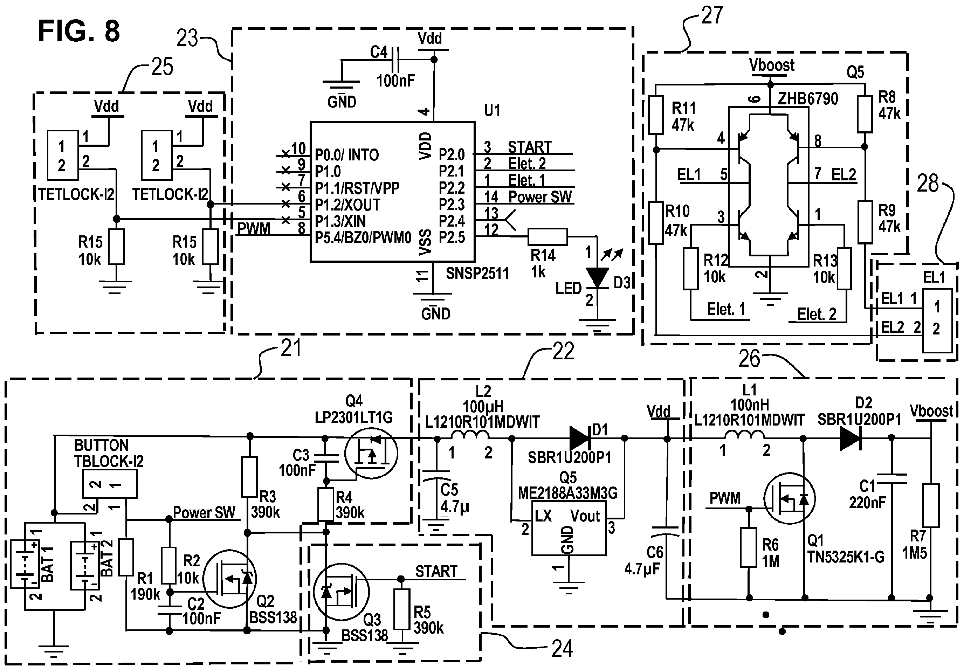

These modules will be explained in details below, together with the description of this innovative electrical equipment, shown in attached Figure 8.

As verified in said Figure 8, the circuit (21), which refers to the Power Supply Module (13), supplies all the other circuits with the voltage supplied by the battery. When MOSFET "Q2" is saturated with the signal coming from the power bottom, it will saturate MOSFET "Q4", energizing the circuit (22), which refers to the Step-up Regulator Module (14).

Circuit (22) has a "Q5" Step-Up Regulator Module, which acquires the voltage of the battery and raises it to 3.3 V. This output voltage is filtered by the capacitor "C6", and then supplied to the circuit (26) and to power the microcontroller.

In the circuit (23), which refers to the Microcontroller Module (15), there is microcontroller "U1", which is responsible for controlling all functions of the equipment. Once it has been powered, the "D3" LED flashes in an intermittent manner to indicate the operation of the equipment. Furthermore, the microcontroller then monitors the signal of the power button and the intensity control buttons, transmits the PWM signal to "Q1", transmits the signals to control the H-Bridge and also trabsmits a signal to "Q3" in the circuit (24), which refers to the Power Seal Module (16), responsible for keeping the MOSFET "Q4" of the circuit (21) saturated, which makes it unnecessary for the Power button to be pressed to allow the equipment to remain energized.

Circuit (25), which refers to the Intensity Regulation Module (17), consists of two buttons controlling the intensity of the electrical stimuli, one button to increase and the other to decrease the intensity. These signals are transmitted to the microcontroller (23) and then the embedded software changes the PWM signal which is transmitted to the circuit (26).

Circuit (26), which refers to Boost Source Module (18) is a boost-type DC/DC converter whose function is to raise the voltage range of 3.3 V to the level that will be used in electrostimulation. The converter is controlled by the intensity regulation buttons of circuit (25) through a PWM signal applied to the MOSFET "Q1", and the output voltage is sent to the circuit (27) through the capacitor "C1 ".

Circuit (27), which refers to a H-Bridge Module (19), has the function of discharging in the electrodes the energy stored in the capacitor "C1" of the boost source. Another feature is to reverse the polarity of current flowing through these electrodes, said inversion occurring according to the digital signals emitted by the microcontroller. The width of the pulses outcoming from the H-bridge is also controlled by the same signal which controls polarity reversal, and then, finally, these stimuli are conducted to the user through the electrodes represented by the block (28) in Figure 8, which refers to the Electrode Output Module (20) in the block diagram of Figure 7.

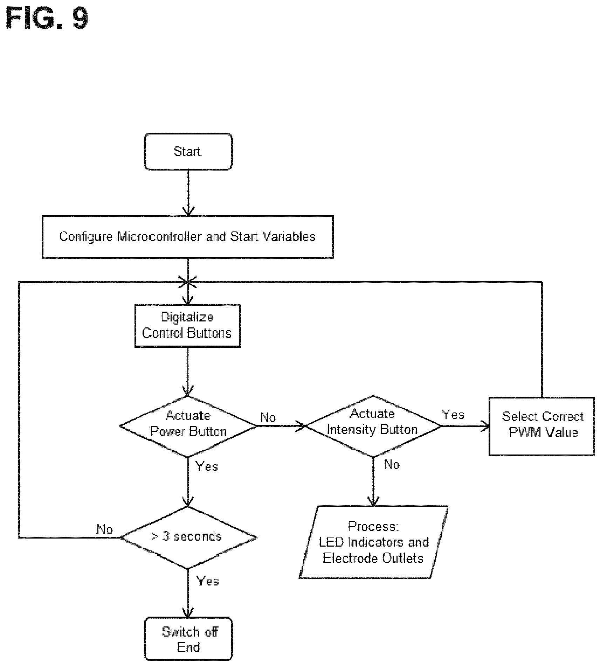

Figure 9 is a flowchart of the software, specifically developed for this innovative muscular electrostimulation protocol.

As already mentioned, this innovative "PORTABLE EQUIPMENT OF ELECTROSTIMULATION" is self-applicable, that is, it can be installed by the athlete himself/herself or by a physical activity practitioner in his/her body, in the anatomical regions corresponding to the major muscular groups intensively exercised in the various exercises and sports modalities, without the need of the assistance of a specialized professional (physiotherapist or technician).

The equipment in question has a high degree of robustness and watertightness, depending on the polymer material used to manufacture the external cabinet (1) and the internal electronic unit (2), which houses the electrical circuit (analog and digital circuits) and power supply. Such equipment is therefore highly resistant to electrical and mechanical shocks and moisture, thus providing a longer service life.

All this, together with its low cost, enabled the use of this equipment outdoor (outside outpatient facilities or gyms), spreading its use to the field, in the environments where the exercises, tests or games are performed.

On the other hand, the equipment in question has an ergonomic configuration, as well as a portability superior to the usual portable equipment known in the art.

Due to all the characteristics reported throughout this specification, these innovative portable muscular electrostimulation protocol and the portable muscular electrostimulation equipment are technically and functionally advantageous, standing out from the conventional processes and equipments known so far, and thus deserving the protection conferred by the present patent.

## Claims

1. A PROCESS FOR ESTABLISHING A MUSCULAR ELECTROSTIMULATION PROTOCOL, to be used for muscular performance recovery of users in general, and more specifically, of the ones practicing physical activity and athletes, **characterized in that** it involves the following steps:
- Step 1: Automatic application (via embedded software) of a first subcycle of electrical pulses, whose width ranges between 100 µs and 400 µs, preferably 260 µs, whose frequency ranges between 4 Hz and 12 Hz, preferably 8 Hz, and whose amplitude ranges between 0V and 100V (with 500Ω charge), preferably ranging between 0V and 60V (with 500Ω charge), transcutaneously, for a period ranging from 1 and 20 minutes, to achieve muscular vasodilation (vasodilation phase);
- Step 2: Automatic application (via embedded software) of a second subcycle of electrical pulses, consisting of pulse bursts, whose pulse width ranges between 100 µs and 400 µs, preferably 260 µs, whose frequency ranges between 20 Hz and 40 Hz, preferably 30 Hz, and whose amplitude ranges between 0V and 100V (with 500 Ωcharge), preferably ranging between 0V and 60V (with 500Ω charge), for a period ranging between 1 and 20 minutes, also transcutaneously, for the differential mobilization of muscle fibers of low energy consumption, aiming to restore the stocks of energy substances in all muscle fibers, including in high consumption ones (recovery phase); this subcycle involves an upward-intensity ramp going from 0V (with 500Ω charge) up to the value initially established by the user, for 1 second, followed by a plateau maintaining this maximum amplitude for 4 seconds, followed by a downward ramp descending from the value established by the user down to 0V (with 500 Ω charge) for 1 second; a stimulation pause of preferably 4 seconds is inserted between each pulse burst, said bursts being repeated several times, accounting to a total from 1 to 20; and
- Step 3: Automatic application (via embedded software) of a first subcycle of electrical pulses, whose width ranges between 100 µs and 250 µs, preferably 180 µs, whose frequency ranges between 1 Hz and 3 Hz, preferably 2 Hz, and whose amplitude ranges between 0V and 100V (with 500 Ωcharge), preferably between 0V and 60V (with 500 Ωcharge), transcutaneously, for a period between 1 and 5 minutes, to obtain muscular vasodilation (vasodilation phase);

2. The PROCESS FOR ESTABLISHING A MUSCULAR ELECTROSTIMULATION PROTOCOL, according to claim 1, **characterized in that**, in the three electrostimulation phases of the process (Steps 1, 2 and 3),
the electrical pulses have a square, trapezoidal or triangular shape, preferably trapezoidal, and/or
the electrical pulses have biphasic or monophasic morphology, preferably asymmetrical biphasic.

3. The PROCESS FOR ESTABLISHING A MUSCULAR ELECTROSTIMULATION PROTOCOL, according to claim 1, **characterized in that** it involves electrostimulation cycles of different durations, each one is adapted to the different situation of use in different sports modalities.

4. The PROCESS FOR ESTABLISHING A MUSCULAR ELECTROSTIMULATION PROTOCOL, according to claims 1 and 3, **characterized in that**, in the long stimulation cycle, Step 1 (vasodilation phase) lasts 10 minutes, Step 2 (recovery phase) lasts 10 minutes, and Step 3 (untightening phase) lasts 5 minutes.

5. The PROCESS FOR ESTABLISHING A MUSCULAR ELECTROSTIMULATION PROTOCOL, according to claims 1 and 4, **characterized in that** subcycles and total time are shorter than said long cycle.

6. The PROCESS FOR ESTABLISHING A MUSCULAR ELECTROSTIMULATION PROTOCOL, according to claim 1, **characterized in that**
it involves the use of electrostimulation cycles in combination with dietary supplements, to obtain the maximum recovery effect from athletic performance, and/or that
it involves the use of electrostimulation cycles in combination with drugs, to increase tissue concentration in the stimulated regions of the body.

7. A PORTABLE MUSCULAR ELECTROSTIMULATION EQUIPMENT USING SAID PROTOCOL to be used in the muscular performance recovery of patients in general, and more specifically, of physical activities practitioners and athletes, said equipment (1) being **characterized in that** it consists of a substantially parallelepiped monolithic cabinet (2), within which there is an electronic module (3), consisting of a body having the same shape, formed by two halves (3a) and (3b), between which a printed circuit board (4) is mounted by miniaturized technology, said board carrying the electronic circuit and the microcontroller responsible for the automated control of stimulation parameters; between the halves (3a) and (3b) of the internal electronic module (3), there is a power supply consisting of at least one battery (5) and a main electrode (6), while, extending from one of the distal sides of said cabinet (2) there are two electric conductor cables (7), at whose ends there are two satellite electrodes (8), said three electrodes (6) and (8) having in on one of their faces, more specifically, on their face facing the body of the patient or athlete, self-adhesive gel plates, for application and fixation in the various regions of the body whose muscles will be electrostimulated; an adjustable strip (9) extends from the non-distal edges of the external lower face of the cabinet (2), wherein said strap (9) can be used to secure the equipment (1) around the upper and lower limbs of the patient or athlete; in the external upper face of the cabinet (2), there is a control panel (10) with a power button (11) and two intensity selection buttons (12); the following internal components are also disclosed: Power source module (13), step-up regulator module (14), micro controller module (15), power supply seal module (16), boost source module (18), H-bridge module (19), electrode output module (20).

8. The PORTABLE MUSCULAR ELECTROSTIMULATION EQUIPMENT USING SAID PROTOCOL, according to claim 7, **characterized in that**
said cabinet (2) consists of a monolithic, watertight block made of flexible polymer, and/or that
said cabinet (2) consists of a monolithic, watertight block made of silicone.

9. The PORTABLE MUSCULAR ELECTROSTIMULATION EQUIPMENT USING SAID PROTOCOL, according to claim 7, **characterized in that** said module (3), which houses the printed circuit board (4), consists of a block formed by two watertight halves (3a) and (3b), made of flexible polymer.

10. The PORTABLE MUSCULAR ELECTROSTIMULATION EQUIPMENT USING SAID PROTOCOL, according to claim 9, **characterized in that** said module (3), which houses the printed circuit board (4), consists of a block formed by two watertight halves (3a) and (3b), made of silicone.

11. The PORTABLE MUSCULAR ELECTROSTIMULATION EQUIPMENT USING SAID PROTOCOL, according to claim 9, **characterized in that** said printed circuit board (4) uses the surface-mount technology (SMT).

12. The PORTABLE MUSCULAR ELECTROSTIMULATION EQUIPMENT USING SAID PROTOCOL, according to claim 9, **characterized in that** said battery (5) is a nickel-cadmium (NiCd), a nickel-metal hydride (NiMh), or a lithium-ion (Li-Ion) battery, preferably a lithium-ion one (Li-Ion).

13. The PORTABLE MUSCULAR ELECTROSTIMULATION EQUIPMENT USING SAID PROTOCOL, according to claim 7, **characterized in that** said gel plates are self-adhesive.

14. The ELECTROSTIMULATION PORTABLE EQUIPMENT USING SAID PROTOCOL, according to claim 7, **characterized in that** it comprises the electrical scheme shown in Figure 8.

15. The PORTABLE MUSCULAR ELECTROSTIMULATION EQUIPMENT USING SAID PROTOCOL, according to claim 7, **characterized in that** it comprises the flowchart shown in Figure 9.
